**Europäisches Patentamt**

**European Patent Office**

**Office européen des brevets**

(19)

(11) Publication number: **0 089 351**
**B1**

(12) **EUROPEAN PATENT SPECIFICATION**

(45) Date of publication of patent specification: **17.09.86**

(51) Int. Cl.⁴: **C 12 M 1/04**

(21) Application number: **82902817.4**

(22) Date of filing: **22.09.82**

(86) International application number:
**PCT/FI82/00037**

(87) International publication number:
**WO 83/01071 31.03.83 Gazette 83/08**

(54) **PROCEDURE AND GAS GENERATING UNIT FOR USE IN MICROBIOLOGICAL CULTIVATION.**

(30) Priority: **25.09.81 FI 812986**

(43) Date of publication of application:
**28.09.83 Bulletin 83/39**

(45) Publication of the grant of the patent:
**17.09.86 Bulletin 86/38**

(84) Designated Contracting States:
**AT BE CH DE FR GB LI LU NL SE**

(56) References cited:
**EP-A-0 039 753**
**DE-B-2 705 096**
**DE-B-2 800 437**
**SE-B- 403 492**
**US-A-4 077 846**
**US-A-4 287 306**

**WILLIS A T, Anaerobic Bacteriology: Clinical and Laboratory Practice (3rd Ed.) published 1977 by Butterworths (London) see pp. 1-18.**

The file contains technical information submitted after the application was filed and not included in this specification

(73) Proprietor: **FENNOPORT KY**
**Marjaniementie 36**
**SF-00930 Helsinki 93 (FI)**

(72) Inventor: **MATIKAINEN, Tapio**
**Puolaharju 36**
**SF-00930 Helsinki 93 (FI)**

(74) Representative: **LOUIS, PÖHLAU, LOHRENTZ & SEGETH**
**Ferdinand-Maria-Strasse 6 ·**
**D-8130 Starnberg (DE)**

Courier Press, Leamington Spa, England.

## Description

The object of the present invention is to provide a procedure and apparatus for enabling a suitable gas atmosphere to be created, especially in microbiological cultivation.

Such apparatus is known in prior art by which it is possible to obtain an anaerobic environment suitable for cultivating microorganisms, for instance bacteria. An apparatus of this kind is disclosed e.g. U.S. Patent No. 3,246,959 and in the British Patent No. 1,544,995. Both references disclose an apparatus by which it is possible to produce in an enclosed vessel an anaerobic environment for bacterial culture. The apparatus disclosed by both references cited is largely similar in principle: they are based on the same basic chemistry, the two types of apparatus are greatly similar in appearance, and the idea of operation is quite nearly of the same type in both: gas generation tablets or equivalent have been packed in a tight bag. This bag may be provided with a porous partition, as in the said U.S. Patent, or the gas generation tablets may be packed in a bag made of porous material and enclosed in a tight bag, as in the British Patent.

In commercial products based on said patents there has been packed usually in an impervious foil bag, one tablet of sodium borohydride, which on adding water produces hydrogen, and two tablets of a mixture of sodium carbonate and citric acid, from which water addition elicits carbon dioxide. When using such a bag, its corner is cut open, water is poured into the bag and the water will gradually, within the bag, be absorbed by said tablets. In an enclosed vessel the oxygen is used up as hydrogen and carbon dioxide are formed. As indicator to ascertain the presence of a proper atmosphere is used methylene blue or a biological indicator, in other words a bacterium which grows or fails to grow in the atmosphere in question.

The formation of gas starts as soon as the liquid comes into contact with the gas generation tablet, and this usually happens immediately after liquid addition has been commenced and, generally, before the apparatus has been closed; therefore in order that anaerobic conditions might be attained with full assurance, the tablets have been so dimensioned that gases which are produced are generated in excess.

It is known through DE—A—2 518 282 to use for gas generation, tablets or equivalent which have been coated with an insulating layer or layers resisting the liquids a short time and which for instance during incubation permit the substance therewithin to be activated. It is reported in the said print that with the aid of such tablets an accuracy of 5 to 15% has been achieved with regard to $CO_2$ gas.

From experiments a very great number of substances is known from which it is possible to generate e.g. gases needed in microbiological procedure, such as hydrogen and carbon dioxide for instance. Various tablets have also been prepared which are suitable for generating hydrogen, which as it reacts with oxygen produces water. As a rule, the tablets have been so made that also a shielding gas is formed, usually carbon dioxide $CO_2$.

Generally speaking there are several different practical methods by which an anaerobic volume can be obtained.

In practice nowadays both in scientific research and for instance in examination of foodstuffs such gas mixtures are increasingly needed which have a given, essentially accurate oxygen content (in other words, the oxygen content is within given permissible percent limits). Likewise, in various investigations nowadays an increasing need exists for gas mixtures in which the content of a given gas is pre-determinable with essential accuracy. For instance in studies of the intestinal bacterial flora, one should be able to create a milieu equivalent to that in the human body.

Until now, for instance when a culture environment has been needed with for instance its oxygen content being of a given kind with essentially high accuracy, one has been compelled to use gas mixtures, the consequence being that the procedures take time and require suitable special apparatus and, frequently, highly trained qualified personnel. This often makes the research costly, and the attempts of culture often tend to fail e.g. in conventional microbiological research laboratories.

The present invention aims to eliminate the drawbacks mentioned.

The procedure of the invention is characterized by the features stated in the characteristic features part of claim 1. The features characteristic of the gas generation unit according to the invention are stated in the characteristic features part of claim 4. The features characteristic of the reagent combination of the invention are readable in the characteristic features part of claim 6. The culture vessel employed in the procedure of the invention is characterized by the features stated in the characteristic features part of claim 7.

The procedure of the invention is characterized in that with its aid is obtained a culture environment for microbiological purposes which is essentially accurate as regards the gas atmosphere. This object of the invention is attained by using a culture vessel according to the invention, and suitable prepared gas generation units, which one understands by the aid of data from the tables or equivalent used in the procedure to apply in an appropriate manner, in each case the required number of required units, this number depending on the enclosed volume available, on the number of culture units and on which organism is being cultivated.

The invention is described more closely in the following, referring to the figures of the drawings attached and to the embodiment examples.

Fig. 1 presents in vertical cross-section, a culture vessel in which has been placed a culture rack and therein the culture units, for instance Petri dishes, and in the topmost of these, the gas generation units;

Fig. 2 displays the enclosed culture vessel in vertical cross-section;

Fig. 3 presents the dosage table for the gas generation units.

A culture vessel 1 of standard capacity is selected which is provided with sealing elements or equivalent 11 and can be tightly closed with a preferably transparent cover 10, and in which can be placed racks 2 or equivalent for the culture units 3, such as Petri dishes, test tubes or equivalent.

Of the culture units 3 on the culture rack 2, one unit 4 may be reserved for gas generation purposes, or the gas generation units may be added on the substrate on a base on the bottom of the culture vessel 1. In this unit, 4 or on the base on the bottom of the culture vessel 1 are added the gas generation units, comprising the gas generation reagent in powder form, as tablets, pressed pellets, capsules or equivalent with their base, if any, which base may be coated with a suitable material if necessary. If a catalyst 12 is required in the gas generation process, the catalyst may be placed in a suitable location, for instance mounted in the cover 10 of the culture vessel 1.

It is possible for ascertaining the presence of a proper atmosphere, to provide the culture vessel 1 with a suitable indicator 13, such as a strip of indicator paper or equivalent. It is for instance possible as indicator 13 to use a chemical indicator, a suitable pure culture, or a combination of the preceding.

It is possible to draw up for each microorganism culture a dosage table 14, or equivalent, the data therein contained enabling one to find out directly the quantity of gas generation reagents required for each culture.

In order to achieve transport, storage and easy dispensing, the afore-mentioned gas reagent units may be packaged in suitable packages, for instance in press-out packages.

In the above-mentioned tightly closable culture vessel 1 of standard size are placed the culture unit racks 2, with duly prepared cultures, nutrients etc.

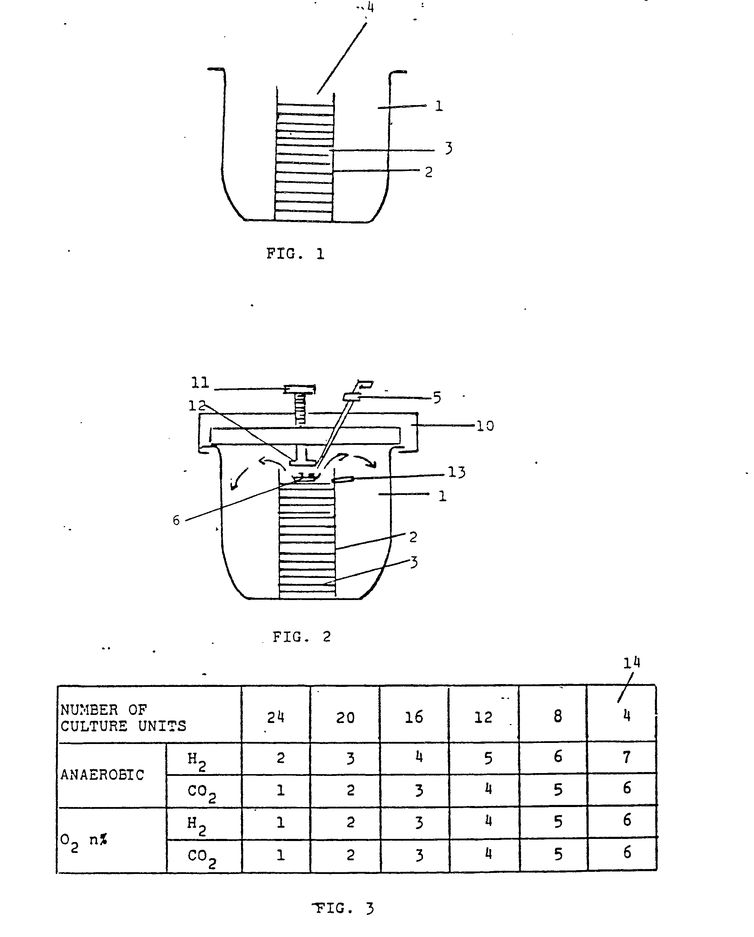

To one culture unit 3—in case a culture vessel 1 of the kind depicted in Fig. 1 or 2 is used, comprising Petri dishes 3 or equivalent stacked in a rack 2—and preferentially to the topmost of them, 4 or on a base on the bottom of the culture vessel 1, is added the quantity of gas generating tablets, pellets, capsules or equivalent needed for gas generation and proper for producing the atmosphere required in the cultivation process.

It is possible to look up in the dosage table 14, for instance how many gas generation tablets should be added into the culture vessel 1 if the full number of culture units 3 has been put in (e.g. 24 units) and if an anaerobic gas atmosphere is desired (e.g. 2 units producing hydrogen and one unit producing carbon dioxide). Likewise, it can immediately be seen in the dosage table 14 how many gas units are required if the full number of culture units has not been entered, or if one desires, instead of an anaerobic space, a certain type of gas environment with regard to oxygen percentage.

For example, when a given microbe is being cultivated which requires a certain kind of atmosphere as regards oxygen and carbon dioxide, one may directly read from Table 14 the number of gas units required, for instance of $H_2$ tablets producing carbon dioxide and hydrogen, if the full number of culture units has been inserted (24 in the example) or if the number of culture units is not complete.

In such case, the oxygen content (in per cent) within the vessel may be controlled within the limits of 21—0%, i.e., from the oxygen content of atmospheric air, 21%, down to anaerobic conditions.

The volumetric capacity of the vessel with standard dimensions is easy to calculate by subtracting from the capacity of the closed space the volume of the contents, which is simply the number of culture substrates times the volume of one substrate.

The culture vessel is tightly closed after the gas units 9 have been added.

The liquid required for gas generation is inserted into the tightly closed vessel e.g. through a suitable valve 5, which may be any kind of gas-tight check valve known in itself in the art and which may have been fitted to the vessel or to its cover, and the valve is at once tightly closed and the liquid wets the gas generation tablets and the gas generation starts.

The procedure of adding the liquid into the closed vessel through a valve or equivalent which is immediately closed to be hermetically sealed enables all the gas that is being formed to be retained in the enclosed space.

According to theoretical calculations, to consume the oxygen from one litre the quantities of sodium borohydride given in the following Table are required:—

TABLE I

| NaBH$_4$ (mg) | O$_2$ consumed % | NaBH$_4$ (mg) | O$_2$ consumed % |
|---|---|---|---|
| 0 | 0 | 93 | 11 |
| 8 | 1 | 102 | 12 |
| 17 | 2 | 110 | 13 |
| 25 | 3 | 119 | 14 |
| 34 | 4 | 127 | 15 |
| 42 | 5 | 136 | 16 |
| 51 | 6 | 144 | 17 |
| 59 | 7 | 153 | 18 |
| 68 | 8 | 161 | 19 |
| 76 | 9 | 169 | 20 |
| 85 | 10 | 178 | 21 |

On the basis of these calculations, pellets were prepared, each containing 185 mg NaBH$_4$, which thus in theory should suffice to eat up the oxygen from a volume of 1 litre, and the following experiments were carried out:—

Experiments with anaerobes:

Using gas generation units as taught by the invention, experiments on growing anaerobic bacteria have been carried out.

For producing hydrogen gas, sodium borohydride pellets according to the invention were used, having the average weight of about 260 mg. whereof 185 mg consisted of the substance in question. For producing carbon dioxide, soda tablet; were used, containing 468 mg of Na bicarbonate per tablet.

Conventional cultivation jars were used for culture vessels.

For catalysts were used palladium-coated aluminium granules. The catalysts were hot-regenerated every time before use. All gas generator tablets were dispensed into the bottom part of an empty bacterial dish of plastic. The requisite water was added. The tests were performed with anaerobic test strains:—

| 1. Bacterioides fragilis No. | 19500/81 |
|---|---|
| 2. Fusobacter necrophorum | ATCC 25286 |
| 3. Fusobacter nucleatum | ATCC 10953 |
| 4. Fusobacter varium | ATCC 8501 |
| 5. Costridium novyl | ATCC 19402 |
| 6. Clostridium tetani | EK 574 II |
| 7. Peptococcus | 2948 |

The aerobic control strain was Pseudomonas aeruginosa. As chemical control for anaerobicity was used an Oxoidin reazurine or BLL-methylene blue strip.

The quantity of carbon dioxide was maintained at the same level (about 8%) throughout the set of test series.

Scrutiny of the culture results obtained revealed that by calculation 1.06 tablets of sodium borohydride were needed per one litre of capacity of the anaerobic jar, corresponding to 196 mg of the substance. It was found that the use of the borohydride pellets was easy and that the required anaerobic conditions could be easily generated with them.

The anaerobic state could be ascertained in the manner known in the art, with the aid of indications, and it was found that it is indicated, as a rule, to apply a slight over-dosage compared with the theoretical values so that not only an anaerobic state might be achieved but that this state might be achieved within a given time.

Aerobic microbes

The invention has a particularly essential significance in the research concerning those microbes which, in order to grow, require an essentially accurate gas atmosphere. With the aid of the invention excellent results have been obtained in studies on intestinal bacteria (cambylo-bacteria), of which the study has been difficult heretofore and repeatable study, almost impossible.

The growth experiments with Cambylo-bacteria were carried out using dosable gas generation units according to the invention and using for hermetically enclosed culture vessel, a culture jar provided with a valve, by which valve the liquid could be dispensed into the enclosed volume.

The cultivation units, catalysts and indicators were introduced into the culture vessel in conventional manner, the gas generation units of the invention were dispensed, the vessel was closed to be air-tight, and the liquid was added with a syringe through the valve.

Reference tests were carried out in a manner known in the art, using a ready-made mixture gas from a gas flask, this gas mixture containing 5% oxygen, 10% carbon dioxide and 85% nitrogen.

The experiments were carried out as follows.

**0 089 351**

| Nutrient substrate | Miller-Hinton+Iso Vitalez agar |
| Growing time | 48—72 hours |

Temperature for various microbes:

| C fetus ss. | intestinalis | +37°C |
| | fetus | +37°C |
| | jejuni | +43°C |

Comparison between the cultures performed as taught by the invention and those according to prior art yielded the following results in a total of 37 trials:—

| better growth (difference 0.2 g or more) with tablets than with gas mixture | in 18 trials |
| equal growth | in 18 trials |
| gas mixture superior | in 1 trial |

In general, equal or better results were obtained with 2—3 $H_2$ tablets and 4 $CO_2$ tablets as/than with gas mixture when cultivating C. jejuni strains. The cultivation modes were compared with each other during three weeks in routine fecal cultures. The number of positive specimens obtained totalled 20, whereof one failed to grow in the gas mixture; otherwise the results were equal regarding the number of findings. Some of the strains failed to grow in larger colonies in some gas environment, others again in other environments, but no regular differences were noted in the growth of Cambylo-bacteria. The nutrient medium employed in the routine cultures was blood agar with five different antibiotics to achieve selectivity.

In addition, the clearly evident circumstance was noted that when using tablets, interfering growth of extraneous bacterial flora would occur to lesser degree than in the gas mixture.

The experiments that were carried out have demonstrated that with the aid of the invention a procedure well applicable in microbe cultivation is obtainable wherein one is enabled, by using constant dosage of gas generation units, to create in an enclosed space an essentially accurate gas atmosphere.

With the aid of the invention a wetting system is achieved which is such that all the gas that is produced remains within the culture vessel.

The gas generation reagents may be condensed in dosage units, tablets, pressed pellets, capsules or equivalent also in such manner that the surface coating protecting them allows absorption of the liquid through the surface layer into contact with the gas generation reagent not before the passage of a given, pre-determined time.

It is also possible to prepare capsules or tablets or equivalent which have been shaped to be "stratified" so that the liquid will penetrate by absorption to the generation reagent through the first protective layer, producing a first atmosphere which is required e.g. for growing a certain microorganism, and after a given time the liquid penetrates through a second protective layer, thus causing a suitable change of the atmosphere so that an atmosphere is obtained which either terminates the growth of the afore-mentioned organism or, for instance, produces an atmosphere which is proper for the growth of another given organism.

It is possible by applying the procedure of the invention, for instance, to simulate the atmosphere within the digestive system and to study for instance the microbial flora of the digestive system. The invention may also be applied in foodstuff studies in studying the keeping quality of foodstuffs.

It is essential that it is possible with the aid of the present invention to obtain a method by which it is possible repeatably to produce gas contents and/or gas mixtures which are substantially conformable as to their content, because the equivalent liquid addition required for generation of water or for other gas generation is made to the gas generation unit already placed in an enclosed space, with suitably high speed through a closable cock or equivalent. It can be experimentally demonstrated that inside the culture vessel for instance the oxygen content can be regulated with an accuracy of about ±1%. The same also applies for instance to carbon dioxide contents, or to methane or any other desired gas.

It is possible by the procedure of the invention to obtain for microbiological cultures, in each instance, the desired gas environment in usual conditions, under normal pressure, without any special equipment, without specially trained personnel. In the procedure of the invention it is possible to utilize normal apparatus employed in microbiological culture operations, such as Petri dishes, flasks, culture racks, etc.; naturally it is feasible in appropriate conditions to evacuate the culture vessel before adding the liquid required for gas generation.

The invention may also be usefully applied in transports which require a given kind of gas environment.

The invention is particularly well applicable in field conditions, where inconvenience, high expenses and loss of time would be incurred in transporting gas flasks and equivalent equipment to the site and in procuring operating personnel.

The procedure in question may also be applied when examining extensive series of samples. The procedure is also applicable in automated microbiological analyzers, and in such case the adding of gas generation units, as well as for instance the monitoring of the indicator revealing the presence of a proper atmosphere, can be automated.

In the drawings only one example has been depicted of the vessel which can be used as

5

taught by the invention. However, the invention is not meant to be confined to this example: the invention may be altered and modified within the scope of the claims.

## Claims

1. Procedure usable in microbiological cultivation, for gas generation, in particular when a given type of gas atmosphere is required, wherein culture units (3), gas generation units to produce said gas atmosphere and, if needed, a catalyst are introduced into a closable vessel (1), characterized in that a number of gas generation units corresponding to a predetermined type of gas atmosphere in the vessel (1) is dispensed into the vessel (1), thereafter the vessel (1) is closed and after closing thereof gas generation is brought about by addition from outside of a liquid, e.g. water, whereby the gas generated all remains in the vessel (1) to essentially accurately create said predetermined gas atmosphere.

2. Procedure according to claim 1, characterized in that the culture vessel (1) is provided with a suitable indicator (13) for ascertaining the presence of a proper atmosphere, which indicator (13) may be chemical or a suitable pure culture.

3. Procedure according to claim 1 or 2, characterized in that in order to obtain said predetermined gas atmosphere within the vessel (1) there is placed a given number of gas generation units with different gas generating parts arranged in layers.

4. Sales unit including a plurality of gas generation units for use in a procedure according to claims 1, 2 or 3, each gas generation unit in the form of a tablet or equivalent hermetically packaged for storage and containing an agent for producing a desired gas atmosphere within a given volume, characterized in that each gas generation unit contains said agent in an amount sufficient to produce a predetermined fraction only of said desired gas atmosphere within said given volume and in that a table (14) for dosage of the gas generation units is provided, whereby the number of gas generation units necessary for obtaining said desired gas atmosphere within said given volume may be readily determined.

5. Sales unit according to claim 4, characterized in that the gas generation unit contains an agent capable, when reacting with a liquid, to consume $O_2$ and/or to produce $CO_2$, and in that the amount of such agent present in said gas generation unit is sufficient to consume a predetermined quantity of $O_2$ from and/or to produce a predetermined quantity of $CO_2$ in a predetermined volume of air.

## Patentansprüche

1. In der mikrobiologischen Züchtung anwendbares Verfahren zur Gaserzeugung, insbesondere wenn eine gegenbene Art von Gasatmosphäre erforderlich ist, bei dem Kultureinheiten (3), Gaserzeugereinheiten zur Erzeugung der genannten Gasatmosphäre und bei Bedarf ein Katalysator in ein verschließbares Gefäß (1) eingebracht werden, dadurch gekennzeichnet, daß eine Anzahl von Gaserzeugereinheiten entsprechend einer vorbestimmten Art von Gasatmosphäre in dem Gefäß (1) in das Gefäß (1) eingegegen werden, das Gefäß (1) verschlossen wird und nach dem Verschließen davon die Gaserzeugung durch Zugabe von außen einer Flüssigkeit, z.B. Wasser, bewerkstelligt wird, wobei alles erzeugte Gas in dem Gefäß (1) verbleibt, um im wesentlichen genau die genannte vorbestimmte Gasatmosphäre zu schaffen.

2. Verfahren nach Anspruch 1, dadurch gekennzeichnet, daß das Kulturgefäß (1) mit einem geeigneten Indikator (13) zur Feststellung des Vorhandenseins einer passenden Atmosphäre versehen ist, welcher von chemischer Art oder eine geeignete Reinkultur sein kann.

3. Verfahren nach Anspruch 1 oder 2, dadurch gekennzeichnet, daß zur Erzielung der genannten vorbestimmten Gasatmosphäre in dem Gefäß (1) darin eine gegebene Anzahl von Gaserzeugereinheiten angeordnet wird, bei ·denen unterschiedliche Gaserzeugerteile in Schichten angeordnet sind.

4. Verkaufseinheit mit einer Mehrzahl von Gaserzeugereinheiten zur Anwendung in einem Verfahren nach den Ansprüchen 1, 2 oder 3, wobei jede Gaserzeugereinheit in Form einer Tablette oder in äquivalenter Form für die Lagerung hermetisch verpackt vorliegt und ein Mittel zur Erzeugung einer gewünschten Gasatmosphäre innerhalb eines gegebenen Volumens enthält, dadurch gekennzeichnet, daß jede Gaserzeugereinheit das genannte Mittel in einer Menge enthält, die ausreicht, um nur einen vorbestimmten Bruchteil der genannten gewünschten Gasatmosphäre innerhalb des gegebenen Volumens zu erzeugen, und daß eine Tabelle (14) für die Dosierung der Gaserzeugereinheiten vorgesehen ist, durch die die zur Erzielung der genannten gewünschten Gasatmosphäre innerhalb des gegebenen Volumens notwendige Anzahl von Gaserzeugereinheiten schnell bestimmbar ist.

5. Verkaufseinheit nach Anspruch 4, dadurch gekennzeichnet, daß die Gaserzeugereinheit ein Mittel enthält, das bei Reaktion mit einer Flüssigkeit im Stande ist, $O_2$ zu verbrauchen und/oder $CO_2$ zu erzeugen, und daß die in der genannten Gaserzeugereinheit vorhandene Menge eines solchen Mittels ausreichend ist, eine vorbestimmte Menge an $O_2$ aus einem vorbestimmten Luftvolumen zu verbrauchen und/ oder darin eine vorbestimmte Menge an $CO_2$ zu erzeugen.

## Revendications

1. Procédé utilisable en culture microbiologique pour la production de gaz, en particulier quand un certain type d'atmosphère gazeuse est désiré, dans lequel sont introduits des éléments de culture (3), des éléments de production de gaz pour la production de ladite atmosphère gazeuse et si nécessaire, un catalyseur dans un récipient qui ferme (1), caractérisé par le fait qu'un nombre d'éléments de production de gaz correspondant à un type prédéterminé d'atmosphère gazeuse dans le récipient (1) est diffusé dans le récipient (1), ensuite le récipient (1) est fermé et après sa clôture, la production de gaz a lieu en ajoutant de l'extérieur un liquide, par ex. de l'eau, le gaz produit restant tout entier dans le récipient (1), afin de produire essentiellement et méticuleusement ladite atmosphère gazeuse prédéterminée.

2. Procédé selon la revendication 1, caractérisé par le fait que le récipient de culture (1) est équipé d'un indicateur approprié (13) pour déterminer a présence d'une atmosphère adéquate, lequel indicateur (13) peut être chimique ou d'une culture pure appropriée.

3. Procédé selon la revendication 1 ou 2, caractérisé par le fait qu'afin d'obtenir ladite atmosphère gazeuse prédéterminée à l'intérieur du récipient (1), il y est placé un certain nombre d'éléments de production de gaz avec des parties generantes de gaz differentes disposées en couches.

4. Unité commercialisable comprenant plusieurs éléments de production de gaz pour utilisation dans un procédé selon la revendication 1, 2 ou 3 chaque élément de production de gaz se présentant sous la forme d'une pastille ou équivalent, emballé hermétiquement pour le stockage et contenant un agent destiné à produire une atmosphère gazeuse désirée à l'intérieur d'un volume donné, caractérisé par le fait que chaque élément de production de gaz contient ledit agent en quantité suffisante pour ne produire qu'une fraction prédéterminée de ladite atmosphère gazeuse désirée à l'intérieur dudit volume donné et par le fait qu'un tableau (14) de dosage des éléments de production de gaz est fourni, le nombre d'éléments de production de gaz nécessaire à l'obtention de ladite atmosphère gazeuse désiré à l'intérieur dudit volume donné pouvant être rapidement déterminé.

5. Unité commercialisable selon la revendication 4 caractérisé par le fait que l'élément de production de gaz contient un agent capable, en réaction avec un liquide, de consommer de l'$O_2$ et/ou de produire du $CO_2$ et par le fait que la quantité d'un tel agent présent dans ledit élément de production de gaz est suffisante pour consommer une quantité prédéterminée de $O_2$ et/ou de produire une quantité prédéterminée de $CO_2$ dans un volume d'air prédéterminé.

FIG. 1

FIG. 2

| NUMBER OF CULTURE UNITS | | 24 | 20 | 16 | 12 | 8 | 4 |
|---|---|---|---|---|---|---|---|
| ANAEROBIC | $H_2$ | 2 | 3 | 4 | 5 | 6 | 7 |
| | $CO_2$ | 1 | 2 | 3 | 4 | 5 | 6 |
| $O_2$ n% | $H_2$ | 1 | 2 | 3 | 4 | 5 | 6 |
| | $CO_2$ | 1 | 2 | 3 | 4 | 5 | 6 |

FIG. 3